# EUROPÄISCHE PATENTANMELDUNG

(11) **EP 2 684 673 A1**
(43) Veröffentlichungstag der Anmeldung: **15.01.2014**
(21) Anmeldenummer: 13175061.4
(22) Anmeldetag: 04.07.2013
(51) Int. Cl.: B29C 49/46, B29C 49/64

(54) **Vorrichtung und Verfahren zum Erwärmen von Kunststoffvorformlingen mit Sterilraum**

(30) Priorität: 13.07.2012 DE 102012106310
(71) Anmelder: Krones AG, 93073 Neutraubling (DE)
(72) Erfinder: Schönberger, Wolfgang, 93073 Neutraubling (DE); Schwöd, Gerhard, 93073 Neutraubling (DE); Winzinger, Frank, 93073 Neutraubling (DE)
(74) Vertreter: Bittner, Bernhard

(57) **Zusammenfassung**

Eine Vorrichtung (1) zum Erwärmen von Kunststoffvorformlingen (10) mit einer Transporteinrichtung (2), welche die Kunststoffvorformlinge (10) entlang eines vorgegebenen Transportpfades transportiert, wobei an dieser Transporteinrichtung (2) wenigstens eine Erwärmungseinrichtung (20) angeordnet ist, welche sich mit den Kunststoffvorformlingen (10) bewegt sowie eine Halteeinrichtung (22) zum Halten der Kunststoffvorformlinge (10), wobei die Vorrichtung (1) einen Reinraum (6) aufweist, durch den die Kunststoffvorformlinge (10) transportiert werden und dieser Reinraum (6) den Transportpfad der Kunststoffvorformlinge (10) umgibt und wobei die Erwärmungseinrichtung (20) einen Aufnahmeraum (31) zum Aufnehmen der Kunststoffvorformlinge (10) aufweist, der die Kunststoffvorformlinge (10) während ihrer Erwärmung wenigstens abschnittsweise umgibt. Erfindungsgemäß weist die Vorrichtung (1) eine Bewegungseinrichtung (25) auf, um die Kunststoffvorformlinge (10) in den Aufnahmeraum (31) einzuführen und aus diesem auszuführen, wobei wenigstens ein Bestandteil (24) dieser Bewegungseinrichtung (25) außerhalb des Reinraums (6) angeordnet ist und eine Dichtungseinrichtung (29,86) vorgesehen ist, welche den Reinraum (6) gegenüber einer Umgebung (U) abdichtet.

## Beschreibung

Die vorliegende Erfindung bezieht sich auf eine Vorrichtung zum Erwärmen von Kunststoff-vorformlingen. Im Stand der Technik ist es bekannt, dass Kunststoffbehältnisse dadurch her-gestellt werden, dass zunächst Kunststoffvorformlinge erwärmt und anschließend beispielsweise mit einer Streckblasmaschine zu den Kunststoffbehältnissen umgeformt werden.

Für viele Getränke ist es erforderlich, diese unter aseptischen Bedingungen abzufüllen. Dabei ist bekannt, dass ein Sterilisationsprozess vor der Blasmaschine eventuell keimbehaftete Kunststoffvorformlinge sterilisiert. Danach kann über eine aseptische Blasmaschine das Behältnis dem aseptischen Abfüllen übergeben werden. Diese Sterilisation der Kunststoffvor-formlinge direkt vor der Blasmaschine bringt gewisse Nachteile mit sich, beispielsweise die restlose Entfernung eines Sterilisationsmittels vor dem Umformungsprozess, jedenfalls jedoch vor dem Füllprozess. Daher wäre es wünschenswert, wenn es möglich wäre, dieser Blasmaschine bereits sterile und von Sterilisationsmitteln freie Kunststoffvorformlinge zuzuführen.

Der vorliegenden Erfindung liegt daher die Aufgabe zugrunde, die Kunststoffvorformlinge bereits zu einem früheren Zeitpunkt steril zu halten und insbesondere bereits in einem sterilen Zustand zu erwärmen. Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Erwärmen von Kunststoffvorformlingen weist eine Transporteinrichtung auf, welche die Kunststoffvorformlinge entlang eines vorgegebenen Transportpfades transportiert. Dabei ist an dieser Transporteinrichtung wenigstens eine Erwärmungseinrichtung angeordnet (und wird daher ebenfalls entlang des Transportpfades transportiert), welche sich mit den Kunststoffvorformlingen bewegt, sowie eine Halteeinrichtung zum Halten der Kunststoffvorformlinge, wobei die Vorrichtung einen Reinraum aufweist, durch den die Kunststoffvorformlinge transportiert werden.

Dieser Reinraum umgibt den Transportpfad der Kunststoffvorformlinge und weiterhin weist die Erwärmungseinrichtung einen Aufnahmeraum zum Aufnehmen der Kunststoffvorformlinge auf, der die Kunststoffvorformlinge während ihrer Erwärmung wenigstens abschnittsweise umgibt.

Erfindungsgemäß weist die Vorrichtung eine Bewegungseinrichtung auf, um die Kunststoffvorformlinge in den Aufnahmeraum einzuführen und aus diesem Aufnahmeraum auszuführen, wobei wenigstens ein Bestandteil dieser Bewegungseinrichtung außerhalb des Reinraumes angeordnet ist und eine Dichtungseinrichtung vorgesehen ist, welche den Reinraum gegenüber einer (unsterilen) Umgebung abgrenzt. Bevorzugt ist eine Dichtungseinrichtung vorgesehen, welche die Bewegung der Bewegungseinrichtung gegenüber dem Reinraum abdichtet.

Es wird daher vorgeschlagen, dass bereits die Erwärmung innerhalb eines Reinraumes stattfindet. Damit wird erfindungsgemäß vorgeschlagen, dass auch der Behandlungsraum, in dem die Kunststoffvorformlinge aufgeheizt werden, so gestaltet ist, dass eine Art Reinraum entsteht, der nach außen hin zu einem unsterilen Raum bzw. einer Umgebung angegrenzt wird und dennoch die Einbringung der erforderlichen Prozessbewegungen zulässt, ohne dabei seine Sterilität zu verlieren.

Bei dem Aufnahmeraum handelt es sich bevorzugt um einen Aufnahmeraum bzw. eine Aufnahmekavität, in welche der Kunststoffvorformling einführbar ist und welche den Kunststoffvorformling in einem eingeführten Zustand bevorzugt vollständig in dessen Umfangsrichtung umgibt. Vorteilhaft umgibt dieser Aufnahmeraum die Kunststoffvorformlinge vollumfänglich in denjenigen Bereichen des Kunststoffvorformlings, welche unterhalb dessen Tragring liegen und welche damit erwärmt werden sollen. Bevorzugt ist ein Querschnitt des Aufnahmeraums an eine Geometrie des Kunststoffvorformlings angepasst und weist bevorzugt einen kreisförmigen Querschnitt auf. Der Aufnahmeraum weist daher bevorzugt eine Öffnung auf, über welche der Kunststoffvorformling eingeführt werden kann. Daneben kann der Aufnahmeraum eine zweite Öffnung aufweisen, durch welche hindurch dem Aufnahmeraum ein Sterilisationsmittel zugeführt, oder über die ein Sterilisationsmittel abgeführt werden kann.

Vorteilhaft weist die Vorrichtung ein Druckbeaufschlagungsmittel auf, welches den besagten Reinraum unter einen vorgegebenen Überdruck setzt. Auf diese Weise kann verhindert werden, dass ungewollt Kontaminationen von außen in den Reinraum eindringen.

Bei einer vorteilhaften Ausführungsform weist die Transporteinrichtung einen drehbaren Träger auf, an dem eine Vielzahl von Erwärmungseinrichtungen angeordnet sind. Damit werden die Kunststoffvorformlinge während ihrer Erwärmung vorteilhaft entlang eines kreisförmigen Transportpfades transportiert.

Vorteilhaft ist ein Antrieb dieser Transporteinrichtung außerhalb des Reinraumes angeordnet. Vorteilhaft weist der Reinraum ein ringförmiges oder torusförmiges Profil auf, wobei insbesondere Bereiche der gesamten Vorrichtung, welche sich radial innerhalb des Transportpfades der Kunststoffvorformlinge befinden, auch außerhalb dieses Reinraumes angeordnet sind. Vorteilhaft ist wenigstens eine Wandung, welche den Reinraum begrenzt, ein Bestandteil des Trägers, an dem die Erwärmungseinrichtung angeordnet ist. Vorteilhaft ist eine Vielzahl von Erwärmungseinrichtungen an dem Träger angeordnet.

Bei einer weiteren vorteilhaften Ausführungsform weist die Vorrichtung eine erste Wandung und eine zweite Wandung auf, welche den Reinraum gegenüber einer Umgebung abgrenzen und diese Wandungen sind relativ zueinander beweglich. Weiterhin ist vorteilhaft eine Dichtungseinrichtung vorgesehen, welche die Bewegung der einen Wandung gegenüber der anderen Wandung abdichtet. Vorteilhaft weist dabei diese Dichtungseinrichtung einen umlaufenden Kanal auf, der bevorzugt mit einer Flüssigkeit gefüllt ist. In diese Flüssigkeit taucht ein Bestandteil der zu diesem Kanal relativ beweglichen Wandung ein.

Bei einer weiteren vorteilhaften Ausführungsform ist wenigstens ein Erwärmungselement zur Erwärmung der Kunststoffvorformlinge außerhalb des Reinraums angeordnet. Dabei wäre es denkbar, dass dieses Erwärmungselement die Kunststoffvorformlinge durch eine Grenze des Reinraums hindurch erwärmt. Bevorzugt kann es sich dabei um ein elektrisch betriebenes Erwärmungselement handeln. So wäre es denkbar, dass das Erwärmungselement selbst in einem abgeschlossenen und gegenüber dem Reinraum abgedichteten Gehäuse (und damit ggfs auch geometrisch zumindest teilweise und bevorzugt vollständig innerhalb des Reinraums) angeordnet ist und die Kunststoffvorformlinge durch eine Wandung dieses Gehäuses hindurch erwärmt werden. Auch wäre es möglich, dass das Erwärmungselement eine Mikrowellenquelle aufweist und diese Mikrowellen durch eine Wandung des besagten Gehäuses treten können.

Bei einer weiteren vorteilhaften Ausführungsform sind mehrere und bevorzugt alle Erwärmungselemente außerhalb des Reinraums angeordnet. Bei einer weiteren vorteilhaften Ausführungsform ist jeweils zwischen zwei Erwärmungseinrichtungen, bzw. zwischen Erwärmungskavitäten, in welche die Kunststoffvorformlinge eingeführt werden, wenigstens ein Erwärmungselement vorgesehen. Vorzugsweise sind zwischen den einzelnen Halteeinrichtungen zum Halten der Kunststoffvorformlinge in der Bewegungsrichtung der Kunststoffvorformlinge jeweils ein oder mehrere Erwärmungselemente angeordnet.

Es wird darauf hingewiesen, dass diese Ausgestaltung auch unabhängig von der oben beschriebenen Erfindung Anwendung finden kann, also insbesondere unabhängig von der Anordnung der Bewegungseinrichtung innerhalb oder außerhalb des Reinraums. Die Anmelderin behält sich vor, auf derartige Ausführungsformen ein eigenes Schutzbegehren zu richten.

Bei einer weiteren vorteilhaften Ausführungsform weist die Vorrichtung einen Drehantrieb auf, um den Kunststoffvorformling wenigstens zeitweise während seiner Erwärmung um dessen Längsrichtung zu drehen.

Durch diese Drehung der Kunststoffvorformlinge wird eine möglichst gleichmäßige Erwärmung derselben erreicht. Damit ist vorteilhaft der Kunststoffvorformling in die besagte Erwärmungseinrichtung bzw. einen von der Erwärmungseinrichtung ausgebildeten Hohlraum einführbar und vorteilhaft auch innerhalb dieses Hohlraumes drehbar, insbesondere um seine eigene Längsrichtung drehbar.

Bei einer weiteren vorteilhaften Ausführungsform ist der Drehantrieb außerhalb des Reinraumes angeordnet.

Bevorzugt weist die Vorrichtung eine Lagereinrichtung zur drehbaren Lagerung der Halteeinrichtung auf. Diese Lagereinrichtung ist dabei insbesondere innerhalb des Reinraumes angeordnet. Dabei ist es möglich, dass die Lagerung einen drehfesten aber insbesondere höhenverschiebbaren, d.h. in der Längsrichtung der Kunststoffvorformlinge verschiebbaren Teil aufweist, sowie auch einen oder mehrere Lagerkörper, welche zur drehbaren Lagerung der Halteeinrichtung dienen. Vorteilhaft ist an dem drehfesten Lagerteil auch der besagte Antrieb zur Drehbewegung angeordnet.

Bei dem Antrieb kann es sich beispielsweise um einen Elektromotor handeln, es wäre jedoch auch möglich, diesen Antrieb über eine Verzahnung zu erreichen. Bei einer weiteren vorteilhaften Ausführungsform ist eine Bewegungseinrichtung in Form eines Motors und insbesondere Linearmotors vorgesehen, welche die Kunststoffvorformlinge in die Erwärmungseinrichtung einführt. Damit bewegt vorteilhaft ein Motor und insbesondere ein Linearmotor auch die Lagereinheit mit der genannten Halteeinrichtung zum Halten der Kunststoffvorformlinge. Vorteilhaft ist die Halteeinrichtung ein Haltedorn, der zum Halten der Kunststoffvorformlinge in diese eingeführt wird. Dabei kann dieser Haltedorn in seiner Umfangsrichtung Spannmittel aufweisen, welche gegen die Innenwandung des Kunststoffvorformlings drücken und damit einen sicheren Halt des Kunststoffvorformlings gewährleisten.

Bei einer weiteren vorteilhaften Ausführungsform weist die Vorrichtung einen stangenartigen Körper auf, der zur Erwärmung der Kunststoffvorformlinge in deren Innenraum einführbar ist. Bei dieser Ausgestaltung wird ein Duplex-Heizverfahren vorgeschlagen, wobei der Kunststoffvorformling bevorzugt gleichzeitig von innen über den besagten stangenartigen Körper und von außen über die Erwärmungseinrichtung bzw. eine Heizbuchse erwärmt wird. Vorteilhaft ist dabei dieses Heizsystem auf einem Rundläufer angeordnet. Diesem werden die Kunststoffvorformlinge bevorzugt zunächst zugeführt, an eine Spannzange oder ein Spannelement zum Halten übergeben und anschließend in die Erwärmungseinrichtung eingetaucht.

Bevorzugt wird parallel hierzu auch der stangenartige Körper bzw. die Heizlanze in den Kunststoffvorformling eingefahren. Der Kunststoffvorformling verweilt nun eine bestimmte Aufheizdauer in diesem Heizsystem, wobei er bevorzugt kontinuierlich in Drehung versetzt wird, um eine gleichmäßige Erwärmung um den Umfang des Kunststoffvorformlings sicher-zustellen. Nach dem Aufheizprozess wird vorteilhaft der Kunststoffvorformling wieder aus der Erwärmungseinrichtung gezogen und bevorzugt an eine Umformungseinrichtung übergeben.

Bevorzugt handelt es sich um ein STIR (selected transmission infrared)- Verfahren.

Bei den hier beschriebenen Ausführungsformen wird insbesondere auch das Halten des Kunststoffvorformlings genauer beschrieben. Die erforderlichen Hubbewegungen der Halteeinrichtung und/oder des stangenartigen Körpers, sowie auch die Einbringung der Rotationsbewegung und die sterile Abdichtung dieser Hub- und Rotationsbewegungen zueinander sowie die Sterilisationsgrenzen des Reinraumes werden hier genauer beschrieben. Vorteilhaft ist die Halteeinrichtung in einem in der Höhe verschiebbaren Beladungskopf (d.h. der oben beschriebenen Lagereinrichtung) gelagert. Somit kann dieser den Kunststoffvorformling über seine Mündung aufnehmen und weiter (z.B. nach unten) in den Reinraum bzw. die Erwärmungseinrichtung eintauchen. Der oben erwähnte stangenartige Körper, der auch hier vorteilhaft in einer linearen Richtung beispielsweise vertikal verschiebbar gelagert ist, kann ebenfalls in den Kunststoffvorformling eingeführt werden.

Vorteilhaft ist eine Bewegungseinrichtung zum Bewegen dieses stangenartigen Körpers außerhalb des Reinraumes angeordnet. Dabei kann ebenfalls wieder eine Dichtungseinrichtung vorgesehen sein, welche diese Relativbewegung gegenüber dem Reinraum abdichtet. Vorteilhaft handelt es sich bei dieser Dichtungseinrichtung um einen Faltenbalg.

Bei einer weiteren vorteilhaften Ausführungsform handelt es sich bei dieser Bewegungseinrichtung um einen Elektromotor und insbesondere einen Linearmotor. Es wäre jedoch auch möglich, andere Antriebe zu verwenden, wie beispielsweise pneumatische oder hydraulische Antriebe. Weiterhin kann auch eine Dichtungseinrichtung vorgesehen sein, welche die Drehbewegung des Kunststoffvorformlings abdichtet.

Bei einer weiteren vorteilhaften Ausführungsform ist die Halteeinrichtung an einem Träger angeordnet, der wenigstens abschnittsweise als Hohlkörper ausgebildet ist. Dabei ist vorteilhaft durch diesen Träger bzw. diesen Hohlkörper der oben erwähnte stangenartige Körper hindurchführbar. Mit anderen Worten kann eine vorteilhaft vertikal verschiebbare und gelagerte Heizlanze über eine hohlgebohrte Halteeinrichtung in den Kunststoffvorformling eintauchen. Bei dieser Ausführungsform ist es bevorzugt möglich, dass eine Drehbewegung mittels eines Rotationsantriebs, der bevorzugt fest an der Halteeinrichtung oder einem Träger angeordnet ist, in diese Halteeinrichtung geleitet wird. Vorteilhaft werden dabei die drehenden Teile dieser Lagereinrichtung über aseptische Lager abgedichtet. Dabei ist bevorzugt wenigstens ein Abschnitt des stangenartigen Körpers auch in den Reinraum einführbar.

Bei einer weiteren vorteilhaften Ausführungsform ist der Drehantrieb für den Kunststoffvorformling außerhalb des Reinraumes angeordnet. Vorteilhaft ist auch eine Dichtungseinrichtung vorgesehen, welche diese Drehbewegung abdichtet. Bei einer bevorzugten Ausführungsform handelt es sich hierbei um eine Dichtungseinrichtung, welche auch eine Linearbewegung, beispielsweise eine Hubbewegung abdichten kann. So wäre es möglich, dass es sich bei dieser Dichtungseinrichtung ebenfalls um ein Wasserschloss bzw. eine hydraulische Dichtung handelt.

Bei der Erwärmungseinrichtung kann es sich insbesondere um einen Infrarotofen oder auch einen STIR-Ofen handeln. Es wäre jedoch auch möglich, dass es sich bei der Erwärmungseinrichtung um eine Mikrowellenerwärmungseinrichtung handelt, d.h. die Erwärmungseinrichtung wenigstens eine Mikrowellenerzeugungseinrichtung (wie insbesondere ein Magneton) aufweist sowie auch einen Resonator, innerhalb dessen die Kunststoffvorformlinge erwärmt werden. Bei dieser Ausführungsform bildet vorteilhaft jede Erwärmungseinrichtung einen derartigen Resonator zum Erwärmen der Kunststoffvorformlinge aus.

Bei einer weiteren vorteilhaften Ausführungsform weist die Vorrichtung eine Sterilisationseinrichtung zum Sterilisieren der Kunststoffvorformlinge und/oder zum Sterilisieren von Innenbereichen des Reinraumes auf. Dabei ist es wie gesagt möglich, dass die Kunststoffvorformlinge selbst sterilisiert werden, es wäre jedoch auch denkbar, insoweit eine CIP-Sterilisations- oder Reinigungseinheit vorzusehen. Dabei kann die Sterilisationseinrichtung derart gestaltet sein, dass sie die gewünschten Bereiche mit einer Strahlung, beispielsweise einer Elektronenstrahlung oder auch einer UV-Strahlung beaufschlagt. Es wäre jedoch auch möglich und bevorzugt, dass zum Sterilisieren bzw. Reinigen ein fließfähiges Medium (beispielsweise Wasserstoffperoxid oder Peressigsäure) eingesetzt wird.

Bevorzugt weist die Sterilisationseinrichtung eine Beaufschlagungseinrichtung auf, welche wenigstens eine innerhalb des Reinraums befindliche Wandung oder eine Begrenzungswandung des Reinraums mit einem fließfähigen Sterilisationsmittel beaufschlagt.

Vorteilhaft weist die Erwärmungseinrichtung neben einer ersten Öffnung, durch welche der Kunststoffvorformling in den Aufnahmeraum eingeführt wird, eine zweite Öffnung auf, welche insbesondere zu Sterilisations- oder Reinigungszwecken dient, beispielsweise hierzu, um ein Sterilisationsmittel der Erwärmungseinrichtung zuzuführen oder von dieser wieder abzuführen.

Bei einer bevorzugten Ausführungsform weist die Beaufschlagungseinrichtung eine Zuführeinrichtung zum Zuführen des fließfähigen Sterilisationsmittels auf, wobei diese Zuführeinrichtung einen Kanal aufweist, der durch wenigstens eine Wandung des Reinraumes verläuft oder aber durch wenigstens einen Bereich der Halteeinrichtung. Bei dieser Ausführungsform kann beispielsweise der Haltedorn bzw. die Halteeinrichtung als Hohlkörper ausgeführt sein. In diesem Falle könnte über die Halteeinrichtung auch ein Sterilisationsmittel in die Kunststoffvorformlinge eingeführt werden. Dabei wäre es insbesondere auch möglich, dieses Sterilisationsmittel während der Erwärmung der Kunststoffvorformlinge einzuführen.

Bei einer weiteren vorteilhaften Ausführungsform können im Inneren des Reinraumes Schrägflächen vorgesehen sein, die ein zielgerichtetes Ableiten eines Sterilisationsmittels ermöglichen.

Die vorliegende Erfindung ist weiterhin auf ein Verfahren zum Erwärmen von Kunststoffvorformlingen gerichtet, wobei die Kunststoffvorformlinge entlang eines vorgegebenen Transportpfades transportiert werden und während dieses Transportes erwärmt werden. Weiterhin werden die Kunststoffvorformlinge zum Zwecke dieser Erwärmung jeweils in diesen Kunststoffvorformlingen zugeordnete Erwärmungseinrichtungen eingeführt, wobei sich auch diese Erwärmungseinrichtungen ebenfalls entlang des Transportpfades bewegen. Weiterhin werden die Kunststoffvorformlinge während ihrer Erwärmung wenigstens abschnittsweise durch einen Reinraum transportiert, wobei dieser Reinraum mittels wenigstens einer Wandung gegenüber einer Umgebung abgegrenzt wird.

Erfindungsgemäß führt eine Bewegungseinrichtung die Kunststoffvorformlinge in einen Aufnahmeraum bzw. eine Erwärmungskavität dieser Erwärmungseinrichtung ein und diese Bewegungseinrichtung ist wenigstens teilweise außerhalb des Reinraumes angeordnet.

Vorteilhaft ist der Transportpfad der Kunststoffvorformlinge ein kreisförmiger Transportpfad.

Weiterhin wird vorteilhaft wenigstens zeitweise wenigstens ein Bereich dieses Reinraumes und/oder der Erwärmungseinrichtung zum Zwecke der Reinigung mit einem Reinigungs-und/oder Sterilisationsmittel beaufschlagt.

Weiterhin werden vorteilhaft die Kunststoffvorformlinge wenigstens zeitweise während ihrer Erwärmung gedreht, insbesondere um ihre Längsrichtung gedreht.

Weitere Vorteile und Ausführungsformen ergeben sich aus den beigefügten Zeichnungen:
Darin zeigen:
   - Fig. 1: eine schematische Darstellung einer Anordnung zum Herstellen von Behält-nissen;
   - Fig. 2: eine Detailansicht einer Erwärmungseinrichtung zum Erwärmen von Kunststoffvorformlingen;
   - Fig. 3: eine Darstellung einer Vorrichtung zum Erwärmen von Kunststoffvorformlingen;
   - Fig. 4: eine Darstellung der Bewegungseinrichtung zum Einführen der Kunststoffvorformlinge in die Erwärmungskavitäten;
   - Fig. 5: eine Detaildarstellung der in Figur 4 gezeigten Darstellung;
   - Fig. 6: eine weitere Darstellung einer erfindungsgemäßen Vorrichtung zum Erwärmen von Kunststoffvorformlingen;
   - Fig. 7: eine weitere erfindungsgemäße Ausführungsform einer Vorrichtung zum Erwärmen von Kunststoffvorformlingen;

   - Fig. 8: eine Darstellung zur Veranschaulichung eines Reinigungsvorganges;
   - Fig. 9: eine weitere Darstellung zur Veranschaulichung eines Reinigungsvorganges; und
   - Fig. 10: eine weitere Darstellung zur Veranschaulichung eines Reinigungsvorganges.

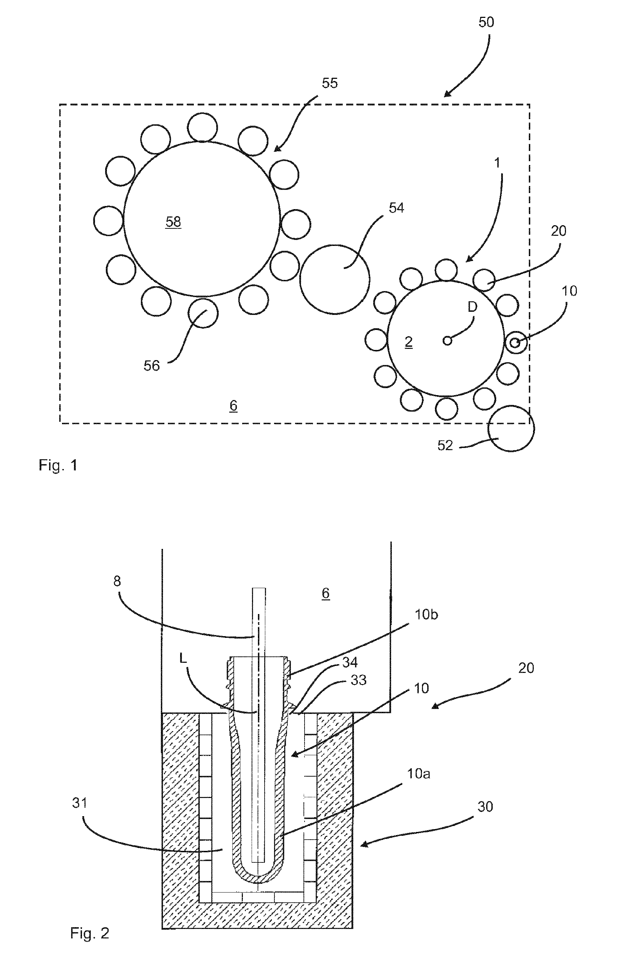

Figur 1 zeigt eine schematische Darstellung einer Anlage 50 zum Behandeln von Behältnissen. Dabei werden Kunststoffvorformlinge 10 (nur einer dargestellt) über eine Zuführeinrichtung 52 einer in ihrer Gesamtheit mit 1 bezeichneten Erwärmungsvorrichtung zugeführt. Diese Erwärmungsvorrichtung weist einen hier einen um eine Drehachse D drehbaren Träger 2 auf, an dem eine Vielzahl von Erwärmungseinrichtungen bzw. -stationen 20 angeordnet ist. Nach ihrer Erwärmung werden die Kunststoffvorformlinge über einen Zuführstern 54 an eine in ihrer Gesamtheit mit 55 bezeichnete Umformungseinrichtung zum Umformen von Kunststoffvorformlingen zu Kunststoffbehältnissen übergeben. Auch diese Umformungseinrichtung weist einen drehbaren Träger 58 und eine Vielzahl von daran angeordneten Umformungsstationen 56 auf. Dabei handelt es sich vorteilhaft bei der Umformungseinrichtung 55 um eine Blasformmaschine und insbesondere um eine Streckblasmaschine.

Sowohl die Erwärmungseinrichtung 1 als auch die Umformungseinrichtung 55 sind hier in einem nur schematisch dargestellten Reinraum 6 angeordnet. Dies bedeutet, dass die Kunststoffvorformlinge bereits während ihrer Erwärmung innerhalb dieses Reinraumes 6 transportiert werden. Weiterhin wäre es denkbar, dass die Anlage beispielsweise Sterilisationseinrichtungen aufweist, welche die Erwärmungseinrichtungen 20 oder auch die Kunststoffvorformlinge 10 selbst sterilisieren. Die Zuführeinrichtung 52 kann dabei ebenfalls zumindest teilweise im Reinraum angeordnet sein.

Figur 2 zeigt eine teilweise Darstellung einer Erwärmungseinrichtung 20. Diese Erwärmungseinrichtung weist eine in ihrer Gesamtheit mit 30 gekennzeichnete Erwärmungskavität auf, welche einen Hohlraum 31 ausbildet, in den der Kunststoffvorformling 10 eingeführt wird. Dieser Kunststoffvorformling 10 weist dabei einen Grundkörper 10a auf, sowie einen Gewindebereich 10b. Dieser Gewindebereich gelangt während der Erwärmung nicht in die Erwärmungskavität 30. Vielmehr ist es denkbar, dass Abschirmeinrichtungen 33 vorgesehen sind, welche eine Erwärmung des Gewindes verhindern. Das Bezugszeichen 34 kennzeichnet eine Öffnung der Erwärmungskavität 30, über die der Kunststoffvorformling 10 der Erwärmungskavität 30 zugeführt werden kann. Jede Abschirmeinrichtung 33 kann dabei als Platte ausgeführt sein, welche wiederum eine Öffnung aufweist, durch welche der Kunststoffvorformlinge 10 hindurchführbar ist.

Das Bezugszeichen 8 kennzeichnet einen stangenartigen Körper, der ebenfalls hier während der Erwärmung in die Kunststoffvorformlinge 10 eingeführt wird. Auch dieser stangenartige Körper 8 erwärmt die Kunststoffvorformlinge 10 von innen. Auf diese Weise ist eine gleichmäßige Erwärmung der Kunststoffvorformlinge 10 möglich. Das Bezugszeichen L kennzeichnet die Längsrichtung der Kunststoffvorformlinge, welche hier mit der Längsrichtung und Bewegungsrichtung des stangenartigen Körpers zusammenfällt.

Das Bezugszeichen 6 kennzeichnet auch hier wieder den Reinraum, innerhalb dessen die Kunststoffvorformlinge erwärmt werden. Da die Erwärmungskavität 30 hier ebenfalls abgeschlossen ist, bildet auch der Hohlraum 31 einen Teil des Reinraumes 6.

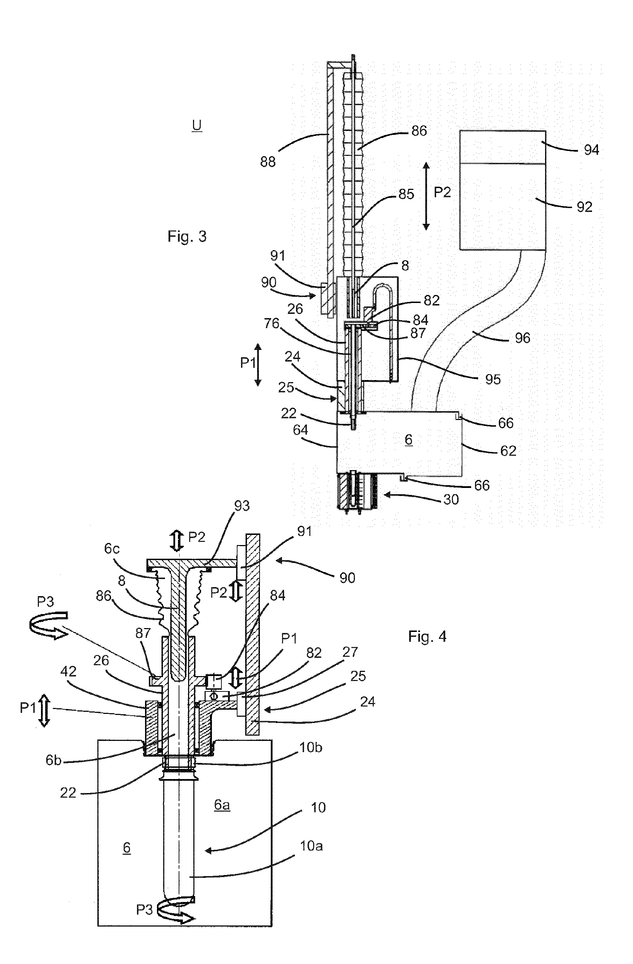

Figur 3 zeigt eine weitere Darstellung der erfindungsgemäßen Vorrichtung. Hierbei ist wiederum die Erwärmungskavität 30 sowie der sich daran anschließende Reinraum 6 erkennbar. Dieser Reinraum 6 ist hier vorteilhaft ringförmig ausgebildet und weist eine erste Wandung 62 sowie eine zweite Wandung 64 auf. Diese zweite Wandung 64 ist dabei beweglich bzw. drehbar angeordnet und die Wandung 62 stationär. Die Bezugszeichen 66 beziehen sich auf Dichtungseinrichtungen, welche die Bewegung zwischen den Wandungen abdichten. Diese Dichtungseinrichtungen 66 können dabei, wie oben erwähnt, jeweils als sogenanntes Wasserschloss ausgeführt sein, wobei ein Abschnitt der Wandung 64 in einen von der Wandung 62 ausgebildeten Kanal eingreift. In dem Sterilraum 6 herrscht dabei bevorzugt ein Überdruck, um ein Eindringen von Keimen zu verhindern. Weiterhin ist ein Gebläse 92 vorgesehen, um über eine Leitung 96 den Sterilraum 6 zu versorgen. Das Bezugszeichen 94 kennzeichnet eine Filtereinrichtung, wie etwa einen Hepa-Filter.

Das Bezugszeichen 24 kennzeichnet einen Stator eines Linearmotors 25, der zur Bewegung der Kunststoffvorformlinge in die Erwärmungskavität 30 (sowie zur Bewegung aus der Erwärmungskavität heraus) dient. Das zugehörige bewegliche Teil bzw. Sekundärteil, d.h. der Läufer, ist hier mit dem Bezugszeichen 26 gekennzeichnet. Dieser Läufer 26 ist dabei als Hohlwelle ausgeführt, so dass durch diese Hohlwelle hindurch auch noch der stangenartige Körper 8 in den Kunststoffvorformling eingeführt werden kann.

Das Bezugszeichen 22 kennzeichnet eine Halteeinrichtung zum Halten der Kunststoffvorformlinge 10. Diese Halteeinrichtung ist hier bevorzugt als Haltedorn ausgeführt, welcher in die Mündungen der Kunststoffvorformlinge eingreift und diese so von innen her hält. Es wären jedoch auch Halteeinrichtungen denkbar, welche die Kunststoffbehältnisse von aussen her greifen. Es wäre jedoch auch denkbar, dass der stangenartige Körper 8, der in die Kunststoffvorformlinge einführbar ist, unmittelbar an der Halteeinrichtung angeordnet ist. So könnte die Halteeinrichtung als langgestreckter Dorn ausgeführt sein, welcher einen Abschnitt aufweist, der in die Kunststoffvorformlinge einführbar ist sowie einen Abschnitt, der die Kunststoffvorformlinge hält.

Das Bezugszeichen 90 kennzeichnet einen zweiten elektromotorischen Antrieb bzw. das Bezugszeichen 91 dessen Stator. Das Bezugszeichen 88 kennzeichnet den Läufer dieser zweiten Antriebseinrichtung 90. Dieser Läufer 88 bewegt dabei eine Trägerstange 85 ebenfalls in der Richtung P2. Das Bezugszeichen 86 kennzeichnet einen Faltenbalg, der diese Bewegung des Trägers 85 in der Richtung P2 gegenüber der Umgebung U abdichtet. Anstelle der Linearmotoren können auch andere Antriebsarten zum Einsatz kommen wie etwa pneumatische oder hyrdraulische Antriebe oder auch sonstige Elektromotoren. Das Bezugszeichen 82 kennzeichnet einen Antrieb, insbesondere einen Elektromotor, der eine Drehbewegung des Kunststoffvorformlings um dessen Längsachse d.h. die Längsrichtung L erzeugt. Dieser Antrieb 82 kann dabei ein Abtriebsrad 84 aufweisen, welches ein an dem an einen Träger 76 angeordnetes Zahnrad 87 antreibt. An diesem Träger 76 ist wiederum die Halteeinrichtung 22 zum Halten der Kunststoffvorformlinge 10 angeordnet und dreht sich daher mit.

Bei den hier gezeigten Ausgestaltungen wird damit wie oben erwähnt ein Duplex-Heizverfahren vorgestellt, bei dem gleichzeitig von innen über eine Heizlanze und von außen über die Erwärmungskavität bzw. Heizbuchse erwärmt wird. Vorzugsweise ist dieses Heizsystem auf einem Rundläufer angeordnet, wobei diesem die Kunststoffvorformlinge zugeführt, an eine Halteeinrichtung 22 zum Halten übergeben werden und anschließend in die Erwärmungskavität eingetaucht werden. Parallel dazu wird auch der stangenartige Körper 8 in die Kunststoffvorformlinge 10 eingefahren. Der Kunststoffvorformling 10 verweilt nun eine gewisse Aufheizdauer in diesem System und wird dabei bevorzugt kontinuierlich in Drehung versetzt, um eine gleichmäßige Erwärmung um den Umfang des Kunststoffvorformlings sicherzustellen. Nach dem Aufheizprozess wird der Kunststoffvorformling wieder aus der Erwärmungskavität 30 gezogen und an die Umformungseinrichtung 55 übergeben.

Figur 4 veranschaulicht diese Funktionsweise und insbesondere die hierzu vorgesehenen Hubbewegungen. Auch hier ist wieder die Halteeinrichtung 22 zum Halten der Kunststoffvorformlinge vorgesehen. Diese ist an einem Träger 26 angeordnet, der, wie durch den Pfeil P3 veranschaulicht, mit dem Kunststoffvorformling 10 drehbar ist. Das Bezugszeichen 10a kennzeichnet einen Grundkörper des Kunststoffvorformlings 10 und das Bezugszeichen 10b einen Mündungsbereich, in den die Halteeinrichtung 22 eingeführt wird.

Man erkennt, dass der Träger 26 hohl gebohrt ist und an einem in der Höhe verschiebbaren Beladungskopf 42, der hier gleichzeitig als Lagereinrichtung dient, angeordnet ist. Das Bezugszeichen 25 kennzeichnet eine erste Bewegungseinrichtung, die zur Bewegung dieses Beladungskopfes bzw. Trägers 42 vorgesehen ist. Diese Bewegungseinrichtung ist hier als Linearmotor ausgestaltet und weist einen Stator 24 auf, an dem ein Läufer 27 in Richtung des Pfeiles P1, d.h. hier nach oben und unten, beweglich ist.

Die Drehbewegung des Kunststoffvorformlings wird hier durch einen Antrieb 82 erreicht, der ein Abtriebsrad 84 antreibt, welches wiederum ein an dem Träger 26 angeordnetes Zahnrad 87 antreibt. Damit ist auch bei dieser Ausführungsform der Antrieb 82 zum Erreichen der Drehbewegung außerhalb des Reinraumes 6 angeordnet. Der Reinraum 6 erstreckt sich hier von dem unteren Bereich 6a über den Hohlraum 6b des Trägers 26 bis in den Raum 6c, der von der Abdichteinrichtung 86 umgeben wird. Die Abdichteinrichtung 86 ist hier als Faltenbalg ausgeführt. Damit setzt sich der Reinraum 6 hier aus den miteinander in Verbindung stehenden Bereichen 6a, 6b und 6c zusammen.

Auch der stangenartige Körper 8 (der in Wirklichkeit länger ist als in Figur 4 gezeigt) kann entlang der Richtung P2, d.h. nach oben und unten, bewegt werden. Auch zu diesem Zweck ist eine in ihrer Gesamtheit mit 90 bezeichnete Bewegungseinrichtung vorgesehen. Diese Bewegungseinrichtung 90 ist hier ebenfalls als Linearmotor ausgestaltet und weist einerseits wieder den Träger 24 (als Stator) auf, andererseits den Läufer 91, der sich in Richtung P2 bewegt. Das Bezugszeichen 93 kennzeichnet den Träger, an dem der stangenartige Körper 8 angeordnet ist.

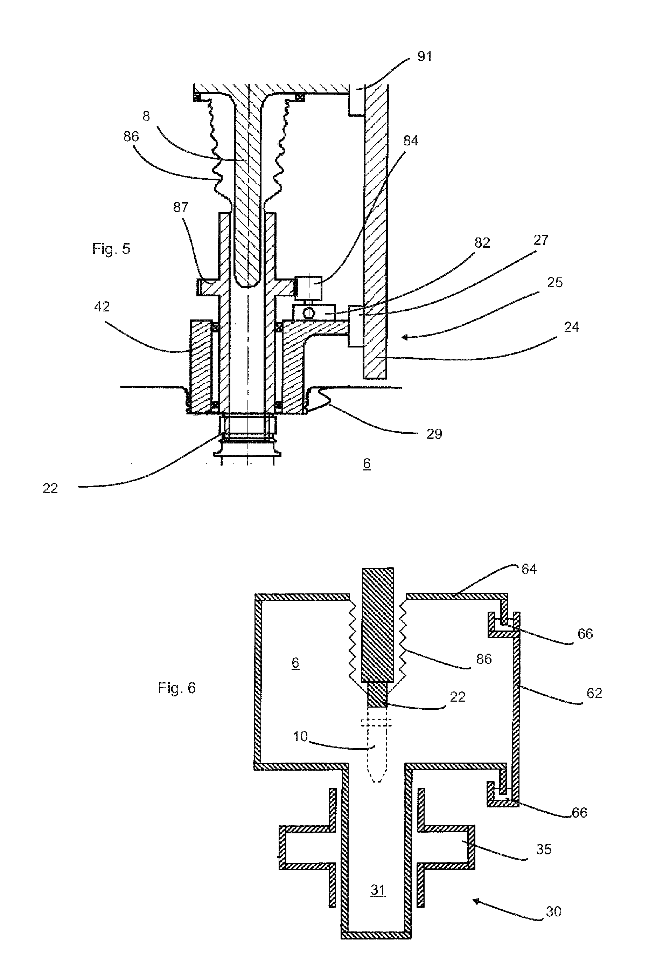

Figur 5 zeigt eine vergrößerte Darstellung der in Figur 4 gezeigten Vorrichtung. Man erkennt hier, dass der Antrieb 82 an dem Träger 42 bzw. dem Beladungskopf angeordnet ist. Weiterhin ist eine weitere Abdichteinrichtung bzw. ein weiterer Faltenbalg 29 vorgesehen, der die Relativbewegung des Trägers 42 bzw. Beladungskopfes in Richtung P1 gegenüber dem darunterliegenden Reinraumabschnitt 6 abdichtet. Die Lagereinrichtung 42 zum Lagern des Trägers 26 ist hier bevorzugt als aseptisches Lager ausgeführt. Dieser Faltenbalg kann beispielsweise einerseits an dem Träger bzw. der Lagereinrichtung 42 und andererseits an einer Wandung des Reinraums (6) angeordnet sein, um so den Übergang zwischen den Reinraumbereichen 6a und 6b (vgl. Fig. 4) abzudichten.

Figur 6 zeigt eine weitere Ausgestaltung einer erfindungsgemäßen Vorrichtung. Hier sind wieder die bezüglich einander bewegbaren Wände 62 und 64 vorgesehen, sowie auch die als Wasserschloss ausgeführte Abdichteinrichtung 66. Ein Faltenbalg 86 ist auch hier wieder vorgesehen, um die Bewegung der Halteeinrichtung 22, an der der Kunststoffvorformling 10 angeordnet ist, abzudichten. Das Bezugszeichen 30 bezieht sich hier wieder in seiner Gesamtheit auf die Erwärmungskavität, wobei hier der Aufnahmeraum 31 zur Erwärmung des Kunststoffvorformlings direkt mit dem Reinraum 6 in Verbindung steht. Das Bezugszeichen 35 bezieht sich wiederum auf eine Erwärmungseinrichtung. Problematisch ist bei dieser Ausgestaltung jedoch eine Drehung des Kunststoffvorformlings, welche von dem Faltenbalg 86 nicht zugelassen wird.

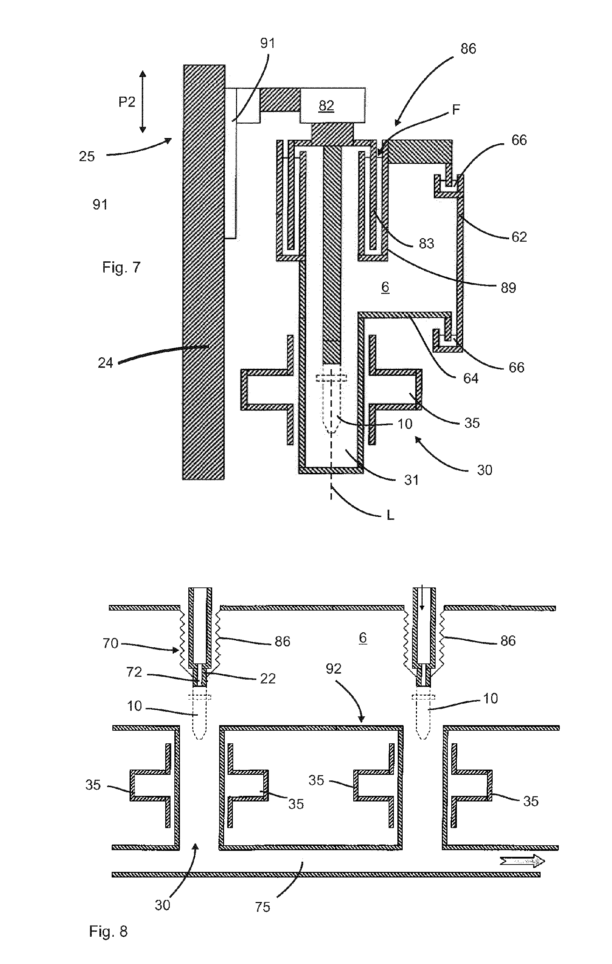

Figur 7 zeigt eine Ausführungsform, bei der auch eine Relativdrehung des Kunststoffvorformlings 10 zugelassen wird. Auch hier ist der Aufnahmeraum 31 vorgesehen, in den der Kunststoffvorformling 10 eintaucht. Als Dichtungseinrichtung 86 ist hier jedoch kein Faltenbalg vorgesehen, sondern ein Wasserschloss, welches sich um die Längsachse L des Kunststoffvorformlings und damit auch um dessen Drehachse herum erstreckt. Dieses Wasserschloss weist dabei einen Aufnahmekanal 89 auf, der wie gesagt kreisförmig und umlaufend ausgebildet ist, sowie ein Schwert 83, welches in eine Flüssigkeit F in diesem Kanal 89 eintaucht. Das Bezugszeichen 91 kennzeichnet wiederum den Läufer, der die Bewegung in Richtung des Pfeiles P2, d.h. die Hubbewegung, durchführt. Das Bezugszeichen 82 kennzeichnet wiederum den Antrieb zum Erreichen der Drehbewegung des Kunststoffvorformlings 10.

Durch die relativ große Höhe der Abdichteinrichtung bzw. des Wasserschlosses 86 ist es hier auch möglich, relativ große Hubbewegungen unter Beibehaltung des Reinraumes 6 zu erreichen.

Wie hier erwähnt, kann eine derartige Vorrichtung zum Erwärmen von Kunststoffvorformlingen mehrere Erwärmungsstationen wie in Figur 7 gezeigt aufweisen. Dabei kann insbesondere auch jede dieser Erwärmungsstationen jeweils eine Abdichteinrichtung 86 in Form eines Wasserschlosses aufweisen. Diese Wasserschlösser können dabei über eine zentrale Verteilung miteinander gespeist werden und es kann vorgesehen sein, dass diese einzelnen Abdichteinrichtungen bzw. Wasserschlösser 86 auch jeweils den gleichen Füllstand mittels einer Überlaufverbindung aufweisen.

Weiterhin kann eine Sensoreinrichtung vorgesehen sein, welche einen Wasserstand der Flüssigkeit F innerhalb des Kanals 89 kontrolliert.

Bei allen hier erwähnten Dichtungseinrichtungen bzw. Dichtungen, die eine Reinraumgrenze bilden, kann es sich auch um Gummidichtungen handeln.

Figur 8 zeigt eine Darstellung der erfindungsgemäßen Vorrichtung in einem Reinigungszustand. Auch hier sind wieder die einzelnen Halteeinrichtungen 22 vorgesehen, an denen die Kunststoffvorformlinge 10 angeordnet sind. Die Heizelemente 35 sind hier jeweils in Abschirmungen 92 angeordnet. Bei den Erwärmungseinrichtungen 30 kann es sich wie oben erwähnt um STIR-Erwärmungseinrichtungen, aber auch etwa um Mikrowellenerwärmungseinrichtungen handeln.

Das Bezugszeichen 70 kennzeichnet hier eine Sterilisations- bzw. Reinigungseinrichtung, wobei hier diese Funktion auch von der Halteeinrichtung 22 übernommen wird. Zu diesem Zweck weist hier die Halteeinrichtung 22 einen Kanal 72 auf, über den ein Reinigungsmittel zugeführt werden kann. Bei der in Figur 8 gezeigten Ausführungsform sind die einzelnen Erwärmungseinrichtungen durch das Gehäuse 92 von dem Reinraum 6 abgeschirmt und damit befinden sich die Erwärmungseinrichtungen 35 außerhalb des Reinraumes.

Durch die Sterilisationseinrichtung 70 ist es möglich, dass eine Innenwandung der Erwärmungskavität 30 kontinuierlich mit einem Reinigungsmedium, beispielsweise mit einem CIP-Medium, umströmt werden kann. Auch ist es möglich, dass mittels des Kanals 72 der Kunststoffvorformling selbst sterilisiert wird.

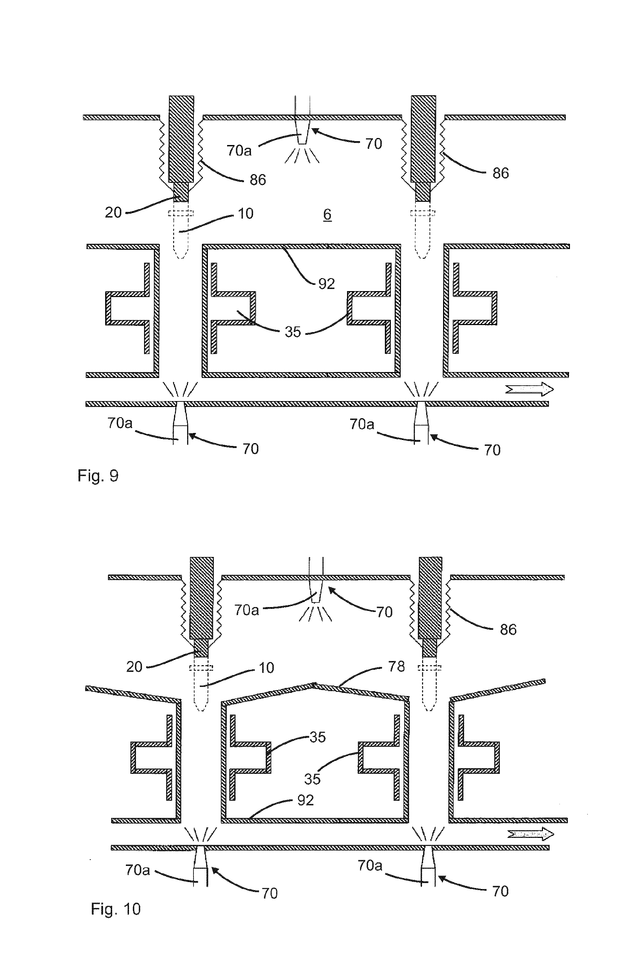

Figur 9 zeigt eine weitere Darstellung einer erfindungsgemäßen Vorrichtung. Auch hier sind wieder Sterilisationseinrichtungen vorgesehen, diese sind jedoch anders als bei der in Figur 8 gezeigten Ausführungsform in den den Reinraum 6 begrenzenden Wandungen untergebracht. Auch bei dieser Ausgestaltung sind die Heizeinrichtungen 35 außerhalb des Reinraumes, in (bevorzugt jeweils abgeschlossenen) Gehäusen 92 untergebracht, angeordnet.

Das Bezugszeichen 75 kennzeichnet eine Abführleitung, über welche Sterilisationsmittel aus der Anlage abgeführt werden kann. Dabei kann eine Pumpe vorgesehen sein, die zur Abführung dieses Sterilisationsmittels dient. Die Bezugszeichen 70a beziehen sich auf Beaufschlagungseinrichtungen bzw. Düsen, welche das Sterilisationsmittel auf innerhalb des Reinraums 6 liegende Bereiche aufbringen.

Bei der in Figur 10 gezeigten Ausführungsform sind die Gehäuse 92 mit Schrägflächen 78 ausgestaltet, so dass Sterilisationsmittel, welches auf diese Schrägflächen gelangt, leichter nach unten hin abfließen kann.

Die Anmelderin behält sich vor, sämtliche der in den Anmeldeunterlagen offenbarten Merkmale einzeln oder in Kombination als erfindungswesentlich zu beanspruchen, falls diese neu gegenüber dem Stand der Technik sind.

### Bezugszeichenliste

- 1: Erwärmungseinrichtung
- 2: Träger/Transporteinrichtung
- 4: Erwärmungseinrichtung
- 6: Reinraum/Sterilraum
- 6a, 6b, 6c: Bereiche des Reinraums 6
- 6b: Hohlraum
- 6c: Raum
- 8: stangenartiger Körper
- 10: Kunststoffvorformling
- 10a: Grundkörper
- 10b: Gewindebereich
- 20: Erwärmungseinrichtung/Reinraum
- 22: Halteeinrichtung
- 24: Stator
- 25: Linearmotor
- 26: Läufer/Träger
- 27: Läufer
- 29: Dichtungseinrichtung
- 30: Erwärmungskavität
- 31: Aufnahmeraum
- 33: Abschirmeinrichtungen
- 34: Öffnung der Erwärmungskavität/Bestandteil
- 35: Heizelemente
- 42: Beladungskopf/Träger/Lagereinrichtung
- 44: Wandung
- 46: Wandung
- 48: Wandung
- 50: Anlage
- 52: Zuführeinrichtung
- 54: Zuführstern
- 55: Umformungseinrichtung
- 56: Umformungsstation
- 58: drehbarer Träger
- 62: erste Wandung
- 64: zweite Wandung
- 66: Dichtungseinrichtung
- 70: Sterilisations-/Reinigungseinrichtung
- 70a: Beaufschlagungseinrichtung, Düse
- 72: Kanal/Drehantrieb
- 75: Abführleitung
- 76: Träger
- 82: Antrieb/Drehantrieb
- 83: Schwert
- 84: Abtriebsrad
- 85: Trägerstange
- 86: Abdichteinrichtung/Wasserschloss/Dichtungseinrichtung
- 87: Zahnrad
- 88: Läufer
- 89: Aufnahmekanal
- 90: zweiter elektromotorischer Antrieb/Antriebseinrichtung
- 91: Läufer des zweiten elektromotorischen Antriebs
- 92: Gebläse/Gehäuse
- 93: Träger
- 94: Filtereinrichtung
- 96: Leitung
- D: Drehachse
- F: Flüssigkeit
- L: Längsrichtung
- P1, P2, P3: Bewegungsrichtungen
- U: Umgebung

## Patentansprüche

1. Vorrichtung (1) zum Erwärmen von Kunststoffvorformlingen (10) mit einer Transporteinrichtung (2), welche die Kunststoffvorformlinge (10) entlang eines vorgegebenen Transportpfades transportiert, wobei an dieser Transporteinrichtung (2) wenigstens eine Erwärmungseinrichtung (20) angeordnet ist, welche sich mit den Kunststoffvorformlingen (10) bewegt sowie eine Halteeinrichtung (22) zum Halten der Kunststoffvorformlinge (10), wobei die Vorrichtung (1) einen Reinraum (6) aufweist, durch den die Kunststoffvorformlinge (10) transportiert werden und dieser Reinraum (6) den Transportpfad der Kunststoffvorformlinge (10) umgibt und wobei die Erwärmungseinrichtung (20) einen Aufnahmeraum (31) zum Aufnehmen der Kunststoffvorformlinge (10) aufweist, der die Kunststoffvorformlinge (10) während ihrer Erwärmung wenigstens abschnittsweise umgibt,
**dadurch gekennzeichnet, dass**
die Vorrichtung (1) eine Bewegungseinrichtung (25) aufweist, um die Kunststoffvorformlinge (10) in den Aufnahmeraum (31) einzuführen und aus diesem auszuführen, wobei wenigstens ein Bestandteil (24) dieser Bewegungseinrichtung (25) außerhalb des Reinraums (6) angeordnet ist und eine Dichtungseinrichtung (29,86) vorgesehen ist, welche den Reinraum (6) gegenüber einer Umgebung (U) abdichtet.

2. Vorrichtung (1) nach Anspruch 1,
**dadurch gekennzeichnet, dass**
die Transporteinrichtung (2) einen drehbaren Träger aufweist, an dem eine Vielzahl von Erwärmungseinrichtungen (20) angeordnet sind.

3. Vorrichtung (1) nach Anspruch 2,
**dadurch gekennzeichnet, dass**
die Vorrichtung (1) eine erste Wandung (62) und eine zweite Wandung (64) aufweist, welche den Reinraum (20) gegenüber einer Umgebung (U) abgrenzen und diese Wandungen relativ zueinander beweglich sind.

4. Vorrichtung (1) nach wenigstens einem der vorangegangenen Ansprüche,
**dadurch gekennzeichnet, dass**
die Vorrichtung (1) einen Drehantrieb (82) aufweist, um den Kunststoffvorformling (10) wenigstens zeitweise während seiner Erwärmung zu drehen.

5. Vorrichtung nach Anspruch 4,
**dadurch gekennzeichnet, dass**
eine Erwärmungselement (35) zur Erwärmung der Kunststoffvorformlinge (10) außerhalb des Reinraums (6) angeordnet ist.

6. Vorrichtung (1) nach Anspruch 5,
**dadurch gekennzeichnet, dass**
die Vorrichtung (1) eine Lagereinrichtung (42) zur drehbaren Lagerung der Halteeinrichtung (22) aufweist.

7. Vorrichtung (1) nach Anspruch 1,
**dadurch gekennzeichnet, dass**
die Vorrichtung einen stangenartigen Körper (8) aufweist, der zur Erwärmung der Kunststoffvorformlinge (10) in deren Innenraum einführbar ist.

8. Vorrichtung (1) nach Anspruch 7,
**dadurch gekennzeichnet, dass**
eine Bewegungseinrichtung (90) zum Bewegen dieses stangenartigen Körpers (8) außerhalb des Reinraums (6) angeordnet ist.

9. Vorrichtung (1) nach wenigstens einem der vorangegangenen Ansprüche,
**dadurch gekennzeichnet, dass**
die Halteeinrichtung (22) an einem Träger (26) angeordnet ist, der wenigstens abschnittsweise als Hohlkörper ausgeführt ist.

10. Vorrichtung nach Anspruch 9,
**dadurch gekennzeichnet, dass**
durch diesen Hohlkörper (26) ein stangenartiger Körper (8) hindurch führbar ist.

11. Vorrichtung (1) nach Anspruch 4,
**dadurch gekennzeichnet, dass**
der Drehantrieb (82) außerhalb des Reinraums (6) angeordnet ist.

12. Vorrichtung (1) nach wenigstens einem der vorangegangen Ansprüche,
**dadurch gekennzeichnet, dass**
die Vorrichtung (1) eine Sterilisationseinrichtung (70) zum Sterilisieren der Kunststoffvorformlinge und/oder zum Sterilisieren von Innenbereichen des Reinraums (6) aufweist.

13. Vorrichtung nach Anspruch 12,
**dadurch gekennzeichnet, dass**
die Sterilisationseinrichtung (70) eine Beaufschlagungseinrichtung (70a, 72) aufweist, welche wenigstens eine innerhalb des Reinraums (6) befindliche Wandung oder eine Begrenzungswandung des Reinraums (6) mit einem fließfähigen Sterilisationsmittel beaufschlagt.

14. Vorrichtung (1) nach Anspruch 12,
**dadurch gekennzeichnet, dass**
die Beaufschlagungseinrichtung (70) eine Zuführeinrichtung (70a, 72) zum Zuführen des fließfähigen Sterilisationsmittels aufweist, wobei diese Zuführeinrichtung einen Kanal (72) aufweist, der durch wenigstens eine Wandung (62, 64) des Reinraums (6) und oder durch wenigstens einen Bereich der Halteeinrichtung (22) verläuft.

15. Verfahren zum Erwärmen von Kunststoffvorformlingen (10), wobei die Kunststoffvorformlinge (10) entlang eines vorgegebenen Transportpfades transportiert werden und während dieses Transports erwärmt werden und wobei die Kunststoffvorformlinge (10) zum Zwecke dieser Erwärmung jeweils in diesen Kunststoffvorformlingen (10) zugeordnete Erwärmungseinrichtungen (20), welche sich ebenfalls entlang des Transportpfades bewegen, eingeführt werden, und wobei die Kunststoffvorformlinge (10) während ihrer Erwärmung wenigstens abschnittsweise durch einen Reinraum (6) transportiert werden, wobei dieser Reinraum (6) mittels wenigstens einer Wandung (62, 64) gegenüber einer Umgebung abgegrenzt wird,
**dadurch gekennzeichnet, dass**
eine Bewegungseinrichtung (25) die Kunststoffvorformlinge (10) in einen Aufnahmeraum (31) dieser Erwärmungseinrichtung (20) einführt und diese Bewegungseinrichtung (25) wenigstens teilweise außerhalb des Reinraums (20) angeordnet ist.
